# EUROPEAN PATENT SPECIFICATION

(11) **EP 1 591 083 B1**
(45) Date of publication and mention of the grant of the patent: **05.09.2007**
(21) Application number: 05009381.4
(22) Date of filing: 28.04.2005
(51) Int. Cl.: A61F 2/38

(54) **Prosthetic knee**
Knieprothese
Prothèse de genou

(30) Priority: 28.04.2004 US 566214 P
(43) Date of publication of application: 02.11.2005
(73) Proprietor: Buechel-Pappas Trust, South Orange, New Jersey 07079 (US)
(72) Inventor: Pappas, Michael J., Caldwell, NJ 07006 (US); Buechel, Frederick F., South Orange, NJ 07079 (US)
(74) Representative: Müller-Boré & Partner Patentanwälte

(56) References cited:
- EP-A- 0 904 748
- US-A- 5 011 496
- US-A- 5 370 701
- US-A- 5 824 096
- US-B1- 6 264 696

## Description

### BACKGROUND OF THE INVENTION

### FIELD OF THE INVENTION

The invention relates to a hinged knee joint prosthesis.

### DESCRIPTION OF THE RELATED ART

A natural knee joint includes the distal end of the femur with articular cartilage, the proximal end of the tibia with articular cartilage and a meniscus between the femur and tibia. The femur and the tibia are held in a proper relationship to the meniscus by ligaments. These stabilizing ligaments include the posterior cruciate ligament, the anterior cruciate ligament and collateral ligaments.

The condyles at the distal end of the femur define compound curves. Hence, the degree of congruency between the condyles of the natural femur and the superior surface of the meniscus varies at different degrees of flexion. Flexion of the knee causes the tibia to rotate relative to the femur about an axis that extends generally in a medial-to-lateral direction. The non-congruent shapes of the femoral condyles and the superior face of the bearing causes the contact area of the femur to roll back relative to the tibia during certain ranges of flexion. Flexion also generates rotation of the tibia about its own axis. The amount of rotation of the tibia during flexion of the knee is controlled and limited by the ligaments.

The natural knee joint can become damaged or diseased. For example, damage or disease to the knee can deteriorate the articular surfaces of the femur or tibia and can damage the articular cartilage between the bones. The prior art includes prosthetic knee joints to replace a damaged or diseased natural knee. A prosthetic knee joint typically includes a femoral component that is mounted to the distal end of a resected femur, a tibial component mounted to the proximal end of a resected tibia and a bearing between the femoral and tibial components. The inferior face of the femoral component of a prosthetic knee joint typically defines a pair of arcuate convex condyles. The superior face of the bearing has regions for articular bearing engagement with the condyles of the femoral component. The superior face of the tibial component may be substantially planar and is engaged with the inferior face of the bearing.

Prior art prosthetic knee joints have taken many different forms, depending upon the preferences of the orthopedic surgeon, the condition of the natural knee and the health, age and mobility of the patient. Some prior art knee joint prostheses fixedly secure the inferior surface of the bearing to the superior surface of the tibial component. Other prior art knee joint prostheses provide somewhat greater mobility and permit rotational movement between the bearing and the tibial component. Still other prior art knee joint prostheses allow even greater mobility and permit a controlled amount of anterior-posterior sliding movement between the bearing and the tibial component in addition to the rotational movement.

Prior art prosthetic knee joints for patients with viable ligaments and good stability have no hinges and rely upon retained ligaments to hold the femoral component in an acceptable range of positions relative to the bearing and the tibial component. However, patients without viable collateral ligaments require a knee joint prosthesis where the femoral component is hinged to substantially prevent both anterior-posterior movement of the femoral component relative to the tibial component and to prevent medial-lateral movement.

U.S. Patent No. 5,824,096 issued to the inventors herein and discloses a hinged knee prosthesis where the condyles of the femoral component are defined by compound curves. Hence, congruency will exist for certain ranges of flexion, but a non-congruent line contact will exist between the femoral component and the bearing during other ranges of flexion. More particularly the femoral component of the prosthesis shown in U.S. Patent No. 5,824,096 is configured to provide a substantially congruent bearing in the critical peak loading phase of the normal walking cycle. However, a reduced posterior femoral radii of the condyles produces a more normal knee motion during flexion with an adequate line contact in deeper flexion phases of non-critical activity. The hinged knee prosthesis of U.S. Patent No. 5,824,096 also permits a controlled range of movement of the bearing relative to the tibial and femoral components along an anterior-posterior axis. Additionally, the hinged connection disclosed in U.S. Patent No. 5,824,096 permits a controlled movement of the femoral component away from the tibial component. The compound curves of the femoral component combined with the ability of the bearing to move in anterior and posterior directions causes the femoral component to climb and roll back on the bearing during certain ranges of flexion. The hinge of the prosthesis shown in U.S. Patent No. 5,824,096 is used primarily for stabilization and performs only a minimal load bearing function. Thus, the hinge can be much smaller than knee prostheses where the hinge performs a primary load bearing function. The smaller hinge minimizes bone removal and hence helps to achieve an improved implant fixation. The smaller hinge also is lighter, and hence minimizes the effect of prosthesis weight on the gait of the patient.

The hinged knee prosthesis of U.S. Patent No. 5,824,096 has performed very well since its first introduction and use. However, wear could be reduced further if femoral congruency existed for a greater range of knee motion, rather than only during peak loading phases during walking. For example, congruent contact during many high load activities, such as stair climbing and decent, arising from a chair and other moderate to deep flexion activities would be helpful for improving wear of the prosthesis. Additionally, a prosthesis with a tibial component that extended a smaller distance into the tibia would require less bone removal for implantation, and hence would be received well.

As a further example, US-5,011,496 relates to a prosthetic knee joint having an extended position, an intermediate position and a flexed position, wherein the motion of the joint includes a minor segment from the extended position to the intermediate position and a major segment from the intermediate position to the flexed position, and wherein the center of pressure between the femoral component and the tibial component moves rearward on the tibia during the minor segment.

US-6,264,696 B1, US-5,370,701 and EP 0 904 748 A2 relate to hinged knee joint prostheses having a tibial component for mounting to a tibia and a femoral component for mounting to a femur, wherein the tibial component and the femoral component are hingedly connected to each other.

Therefore, it is an object of the present invention to provide a hinged knee prosthesis, wherein dislocation is prevented even in situations, where collateral ligaments are insufficient.

This object is fulfilled by a hinged knee prothesis having the features disclosed in claim 1. Two further embodiments are subject of the dependent subclaims.

### SUMMARY OF THE INVENTION

The invention relates to a hinged knee prosthesis with a condylar bearing. The prosthesis includes a femoral component for mounting to the resected distal end of the femur, a tibial component for mounting to the resected proximal end of the tibia, a bearing for disposition between the femoral and tibial components and a hinge assembly for providing stability during articulation between the femoral and tibial components. The prosthesis may further include a patellar component.

The hinged knee prosthesis of the subject invention differs from prior art hinged knee prostheses by providing patellofemoral articulating surfaces that are distinct from the tibiofemoral articulating surfaces. The patellofemoral articulation may be similar or identical to that of prior art knee prostheses and may include a compound curve as the articulating surface on the femoral component. However, the tibiofemoral articulating surface is configured for congruent contact over a larger range of motion than is available for knee prosthesis without distinct tibiofemoral and patellofemoral articulating surface. Preferably the congruent contact for the tibiofemoral articulation extends for substantially the entire range of motion. This provides a significant advantage of reduced wear, as compared to prior art prosthetic knees, including prior art hinged knee prostheses.

Prosthetic knees with distinct patellofemoral and tibiofemoral articulating surfaces have been contemplated in non-hinged prosthetic knee designs. However, such a design would not accommodate valgus-varus motion, which is motion in a generally medial to lateral direction. However, a hinged knee provides adequate resistance to valgus-varus motion, and hence is well suited to distinct patellofemoral and tibiofemoral surfaces.

The tibial component of the prosthetic knee includes an axial support, such as a stabilizing rod that can be mounted in a prepared cavity in the resected proximal end of the tibia. The tibial component further includes a tibial plate that extends transverse to the axial support and that can be mounted on the resected proximal end of the tibia. The tibial plate includes a superior tibial surface that may be substantially planar. The tibial component further includes a conical hole extending through the tibial plate and towards the axial support and stabilization rod. A groove may be formed near the distal end of the conical hole.

The bearing of the prosthetic knee includes a plastic cone that can be mounted in the conical hole of the tibial component so that the bearing can rotate relative to the tibial component. The cone of the bearing preferably includes axial separation means for preventing axial separation or dislocation of the bearing from the tibial component. Thus, the prosthetic knee can provide dislocation resistance at least as good as prior art hinged prostheses, but with a shorter cone and hence less bone removal. The axial separation means may include a groove in the cone of the bearing that will align with the groove in the conical hole of the tibial component when the cone of the bearing is mounted in the conical hole of the tibial component. A snap ring may be engageable in the aligned grooves of the conical hole in the tibial component and in the cone of the bearing. The snap ring prevents the bearing from moving proximally and out of the conical hole in the tibial component. However, the snap ring permits the cone of the bearing to rotate relative to the tibial component. Hence, rotational mobility for the bearing is permitted, but dislocation of the bearing is prevented. Other connections that permit rotation but not separation can be provided

The bearing further includes an inferior bearing surface for bearing engagement on the superior tibial surface of the tibial component. Means may be provided for limiting rotational movement of the bearing on the superior tibial surface. For example, the inferior surface of the bearing may be formed with an arcuate groove, and a pin may project from the superior surface of the tibial component for engagement in the groove of the bearing. Thus, the angular extend of the groove in the bearing will limit the range of pivotal movement of the bearing relative to the tibial component. The bearing further includes a superior condylar bearing region for articular bearing engagement with the femoral component. The bearing further include a hole extending from the proximal or superior end to or towards the distal end. Lower portions of the hole in the bearing may be conically tapered.

The hinge assembly includes a carriage with a shaft configured for insertion into the hole of the bearing. The carriage further includes a head with smoothly polished side surfaces that may be substantially parallel to one another. A hinge support hole extends through the head and transverse to the axis of the shaft. The hinge assembly further includes a hinge pin that can be inserted into the pin support hole in the head of the carriage and a set screw for mounting in a threaded hole in the head to hold the hinge pin in position in the head.

The femoral component includes a superior surface configured for mounting on the resected distal end of the femur. A stabilizing rod may project from the superior surface for mounting in a cavity prepared in the resected distal end of the femur. The femoral component further include hinge support walls that project proximally from the superior surface of the femoral component. The walls are substantially parallel to one another and are spaced apart sufficiently for receiving the head of the carriage. The hinge support walls further include holes that align with one another for receiving opposite ends of the hinge pin. The inferior region of the femoral component include a tibiofemoral articular surface and a distinct patellofemoral articulating surface. The patellofemoral articulating surface may define a curved shape similar to the shape of the articular surface on the femoral component disclosed in the above-referenced U.S. Patent No. 5,824,096. The tibiofemoral articulating surface is configured for congruent articular bearing engagement with the condylar bearing region of the bearing.

The congruent articular bearing engagement of the tibiofemoral articular surface of the femoral component with the condylar bearing region on the superior surface of the bearing provides congruency through virtually all ranges of motion from a slightly hyperextended condition to deep flexion. Thus, the prosthetic knee of the subject invention provides congruent contact during many high load activities, such as stair climbing and descent and arising from a chair. Additionally, the prosthetic knee of the subject invention provides very good resistance to valgus-varus moments. Furthermore, the engagement of the cone of the bearing in the conical hole of the tibial component resists dislocation and the engagement of the stop pin with the groove in the inferior bearing surface of the bearing controls and limits the range of permissible rotation of the tibia relative to the femur. Thus, the prosthetic knee of the subject invention is well suited for those situations where the ligaments are not present or are ineffective.

### BRIEF DESCRIPTON OF THE DRAWINGS

FIG. 1 is a longitudinal cross-sectional view of the assembled and implanted hinged knee prosthesis of the subject invention.

FIG. 2 is a top plan view of the tibial component.

FIG. 3 is a cross-sectional view taken along line 3-3 in FIG. 2.

FIG. 4 is a side elevational view of the bearing.

FIG. 5 is a top plan view of the bearing.

FIG. 6 is a cross-sectional view taken along line 6-6 in FIG. 5.

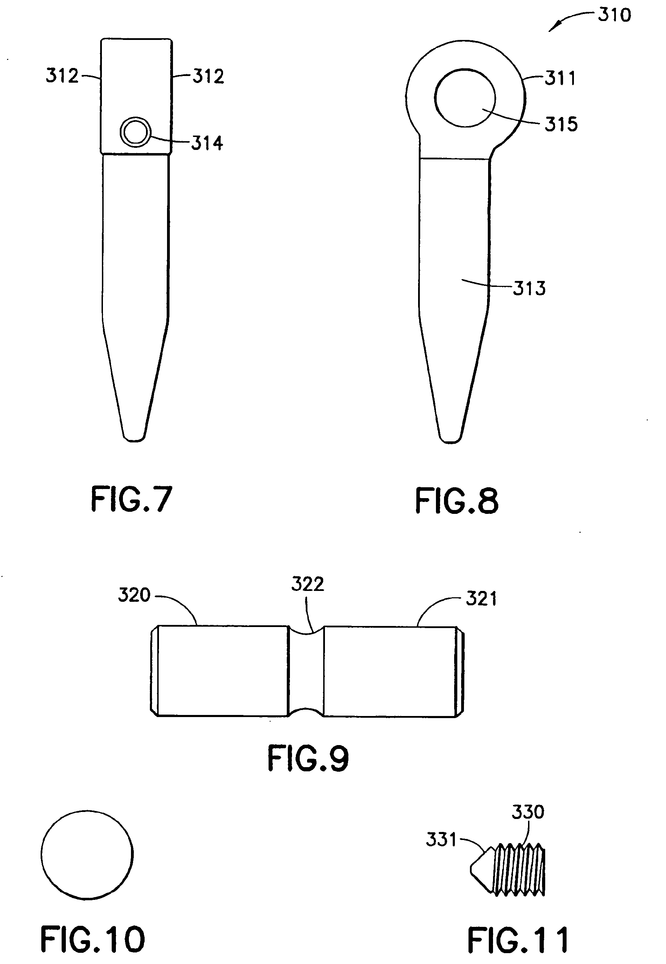

FIG. 7 is a front elevational view of the carriage of the hinge assembly.

FIG. 8 is a side elevational view of the carriage.

FIG. 9 is a front elevational view of the hinge pin of the hinge assembly.

FIG. 10 is a side elevational view of the hinge pin.

FIG. 11 is a side elevational view of the set screw.

FIG. 12 is a rear elevational view of the femoral component.

FIG. 13 is a side elevational view of the femoral component.

FIG. 14 is a front elevational view of the hinge bearing.

FIG. 15 is a side elevational view of the hinge pin bearing.

FIG. 16 is a longitudinal cross-sectional view of the tibial component, bearing and carriage in their assembled condition.

FIG. 17 is a cross-sectional view similar to FIG. 16, but showing movement required for dislocation of the assembled bearing and carriage.

FIG. 18 is a cross-sectional view similar to FIGS. 16 and 17, but showing the movement required for dislocation of the carriage relative to the assembled tibial component and bearing.

FIG. 19 is a rear elevational view of the assembled knee prosthesis.

FIG. 20 is a front elevational view thereof.

FIG. 21 is a side elevational view of the assembled prosthesis showing the knee in a 5° hyperextension.

FIG. 22 is a cross-sectional view showing the assembled knee at approximately 150° flexion, and showing the set screw in an exploded orientation.

FIG. 23 is a top plan view showing relative positions of the patella and femoral component during flexion.

### DETAILED DESCRIPTION OF THE PREFERRED EMBODIMENTS

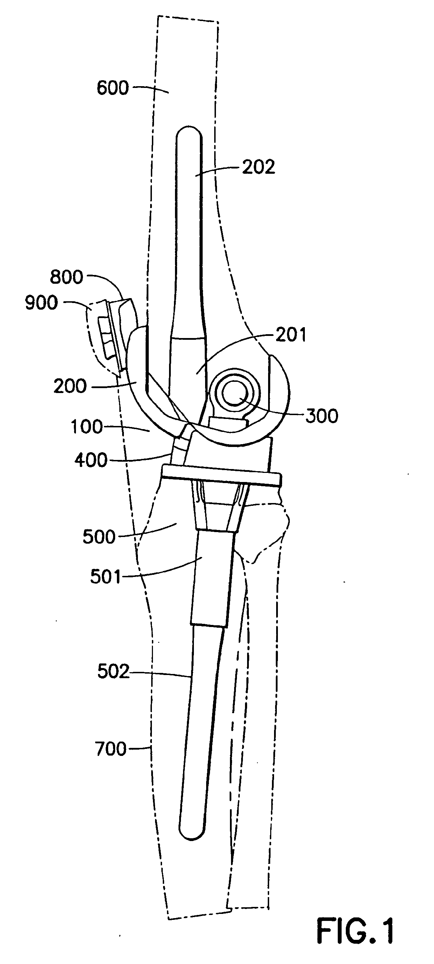

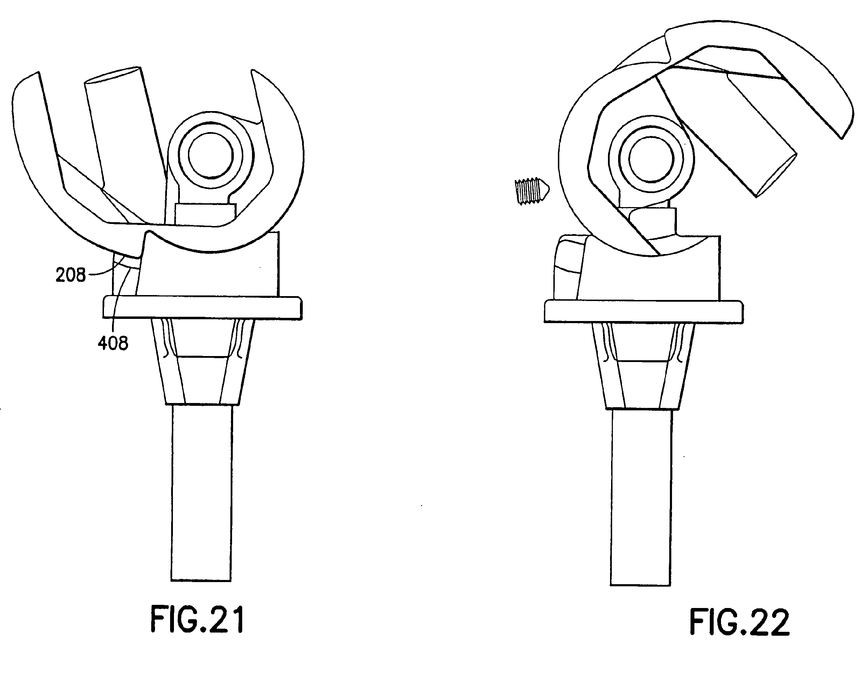

A hinged knee prosthesis in accordance with the invention is identified generally by the numeral 100 in FIGS. 1 and 19-23. The knee prosthesis 100 includes a femoral component identified generally by the numeral 200 in FIG. 1. The femoral component 200 is configured for mounting to the resected distal end of the natural femur 600. The femoral component 200 is configured for articulation relative to an assembly that includes a hinge subassembly 300, a plastic bearing 400 and a tibial component 500. The tibial component 500 is configured for mounting in the resected proximal end of the natural tibia 700. The prosthesis 100 may further include a patellar component 800 that may be implanted in the natural patella 900.

The tibial component 500 includes a body 501 and a stabilizing rod 502 as shown in FIG. 1. Both the body 501 and the stabilizing rod 502 are formed from a metallic material that will provide appropriate strength and biocompatibility. A preferred tibial component 500 is made from a titanium alloy with a TiN coating. However, the tibial component 500 may also be formed from a CoCr alloy. Other metallic materials appropriate for use in the manufacture of the tibial component 500 will be known to those skilled in the art.

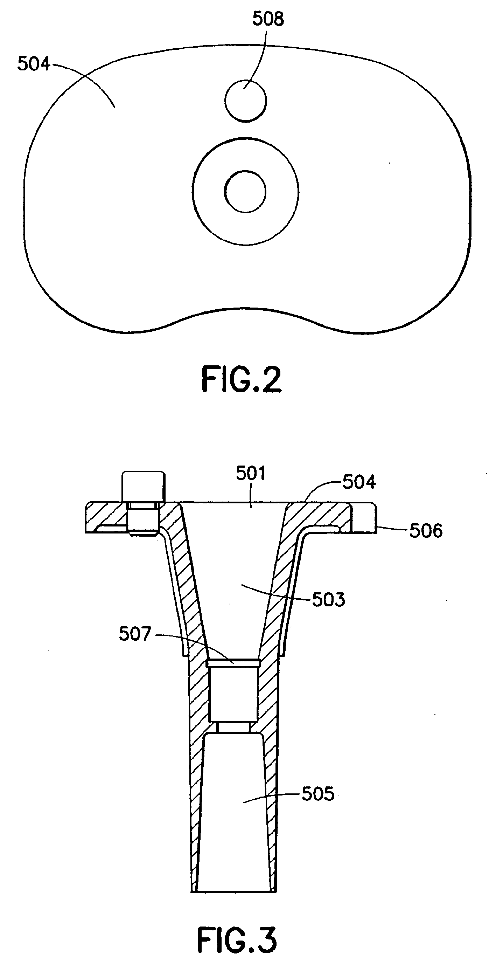

The body 501 of the tibial component 500 is illustrated most clearly in FIGS. 2 and 3. The body 501 includes a conical hole 503 that extends distally from a superior tibial surface 504, and that tapers to smaller dimensions at locations further from the superior tibial surface 504. The conical hole 503 communicates with a cavity 505 that receives the proximal end of the stabilizing rod 502.

A tibial plate 506 extends transverse to the axis defined by the conical hole 503 and includes the superior tibial surface 504. The inferior face of the tibial plate 506 is mountable on the resected proximal end of the natural tibia 700 as shown in FIG. 1. Additionally, portions of the tibial component 500 below the tibial plate 506 are implanted into a cavity prepared in the resected proximal end of the tibia 700. The tibial component 500 may be used with a bone cement to achieve secure anchoring of the tibial component 500 in the tibia 700. Alternatively, external surface regions of the body 501 near the plate 506 may have a bone ingrowth surface region that will encourage growth of the natural bone for secure anchoring of the tibial component 500.

The body 501 of the tibial component further includes an annular groove 507 formed near the distal end of the conical hole 503. Additionally, the tibial component 500 includes a stop pin 508 mounted to an anterior portion of the tibial plate 506. As explained further herein, the stop pin 508 cooperates with the bearing 400 to limit relative rotation between the bearing 400 and the tibial component 500.

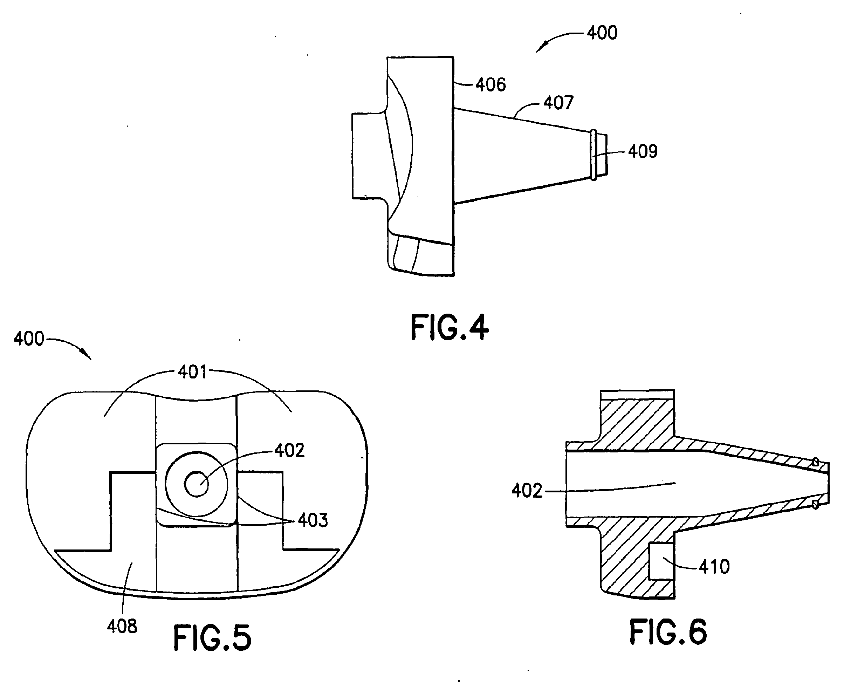

The bearing preferably is formed unitarily from a non-metallic material and most preferably from UHMWPe. The plastic of the bearing performs well under loads, exhibits good biocompatibility and does not interact with the metallic materials of the prosthesis 100 that are adjacent the bearing 400. The bearing 400 includes superior condylar bearing surfaces 401 at medial and lateral positions on the bearing 400. A hole 402 extends in a proximal to distal direction through the bearing 400. As shown most clearly in FIG. 6, the hole 402 includes a generally cylindrical proximal portion and a conically generated distal portion. Proximal portions of the hole 402 extend through a portion of the bearing 400 that is formed with opposite substantially planar side surfaces 403. The side surfaces 403 extend generally in anterior-to-posterior directions and are approximately parallel to one another. The bearing further includes an inferior bearing surface 406 configured for congruent bearing engagement with the superior tibial surface 504. In the illustrated embodiment, both the superior tibial surface 504 and the inferior bearing surface 406 of the plastic bearing 400 are substantially planar.

A cone 407 extends distally from the inferior bearing surface 406 and has an outer surface configured for substantially congruent engagement in the conical hole 503 of the tibial component 500. Thus, the bearing 400 can rotate relative to the tibial component about the central axis of the conical hole 503 in the tibial component 500. This rotation will cause the inferior bearing surface 406 of the bearing 400 to rotate in engagement with the superior tibial surface 504. An anterior and superior position of the bearing 400 includes stop surfaces 408 that limit rotation of the femoral component 200 relative to the bearing in a hyperextension direction, as explained below. An annular groove 409 is formed in the cone 407 of the bearing 400 near the distal end of the cone 407. The groove 409 is disposed to align with the groove 507 of the tibial component 500. A snap ring then can be engaged simultaneously in the groove 409 and 507 to retain the cone 407 of the bearing in the conical hole 503 in the tibial component 500. This engagement will permit rotation of the bearing 400 relative to the tibial component 500, but will prevent dislocation of the bearing 400 from the tibial component 500.

The bearing 400 further includes an arcuate slot 410 formed in an anterior portion of the inferior bearing surface 406. The slot 410 is configured to engage the stop pin 508 of the tibial component 500 and extends through an arc of preferably about 30°. The engagement of the stop pin 508 in the slot 410 limits the range of rotational motion of the bearing 400 relative to the tibial component. The size of the slot 508 and hence the range of rotational movement of the bearing 400 relative to the tibial component 500 will be selected in accordance with the mobility of the patient. In some instances, the slot 410 can be replaced by a cylindrical opening to prevent all rotation between the bearing 400 and the tibial component 500.

The hinge subassembly 300 includes a metal carriage 310 formed unitarily from a sufficiently strong biocompatible material, such as the material used to form the body 501 of the tibial component 500. The metal carriage 310 includes a head 311 with opposite planar highly polished surfaces 312. A shaft 313 extends distally from the head 311. The shaft 313 of the preferred embodiment includes a substantially cylindrical proximal portion and a conically tapered distal portion. The shaft 313 of the carriage 310 is configured for rotational engagement in the hole 402 of the bearing 400. A threaded hole 314 extends through the head 311 from an outer surface region substantially adjacent the shaft 313 to a pin support hole 315 formed in the head 311.

The hinge subassembly 300 further includes a hinge pin 320 configured for engagement in the pin support hole 315. The hinge pin 320 include cylindrical bearing surfaces 321 adjacent opposite longitudinal ends and an engagement groove 322 between the cylindrical bearing surfaces 321..

The hinge subassembly 300 further includes a set screw 330 with a conical leading end 331. The set screw 330 can be threadedly engaged in the threaded hole 314 of the carriage 310 so that the leading end 331 of the set screw 330 engages in the groove 322 of the hinge pin 320. Thus, the hinge pin 320 can be retained fixedly in the pin support hole 315 of the carriage 310.

The femoral component 200 is formed from a metallic material that exhibits sufficient strength and biocompatibility. For example, the femoral component 200 may be formed from the same material described above for the tibial component 500. The femoral component includes a femoral body 201 with a superior surface and a stabilizing rod 202 that extends proximally from the superior surface of the femoral body 201. The femoral body 201 can be mounted to the resected distal end of the natural femur 600 so that the stabilizing rod 202 can be mounted in a cavity prepared in the resected proximal end of the femur 600. The femoral component 200 can be affixed in the femur by bone cement or by natural bone ingrowth that may be promoted by an appropriate external surface configuration on portions of the femoral component 200.

Inferior regions of the femoral body 201 define tibiofemoral articular surfaces 203 that are configured for congruent articular bearing engagement with the condylar bearing surfaces 401 of the bearing 400. More particularly, the tibiofemoral articular surfaces 203 are configured for congruent bearing articular engagement with the condylar bearing surface 401 of the bearing through a broad range of flexion extending at least from full extension to most ranges of flexion that are likely to be generated during high load conditions, such as stair climbing or standing from a sitting position. In a preferred embodiment, congruency will exist from approximately 5° hyperextension to approximately 150° flexion. This congruency results in reduced stress as compared to a line contact or point contact that might be achieved with non-congruent articulating surfaces. As a result, the load is distributed over a wider area and failure during high load activities is much less likely.

Superior regions of the femoral body 201 include spaced apart hinge support walls 204 that are distanced from one another appropriate amounts for receiving the outer side surfaces 312 of the head 311. The superior face of the femoral body 201 also includes a rod support 205 that is engagement with the stabilizing rod 202 of the femoral component 200.

Holes 206 extend through the hinge supports 204 and substantially align with one another. Plastic bushings 210 are engageable in the holes 206 and define internal diameters appropriate for rotatably bearing engaging the hinge pin 320.

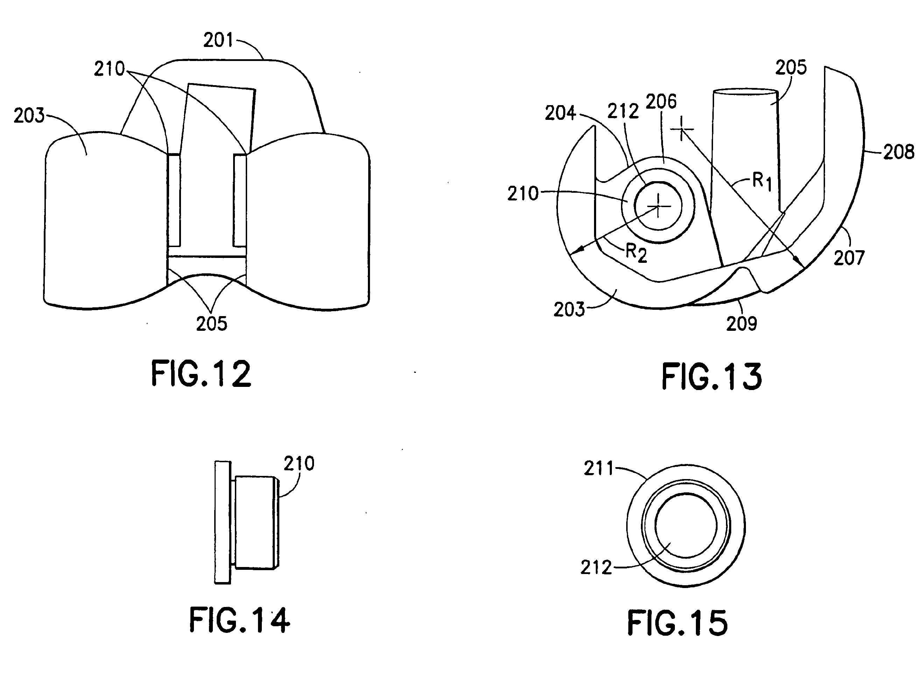

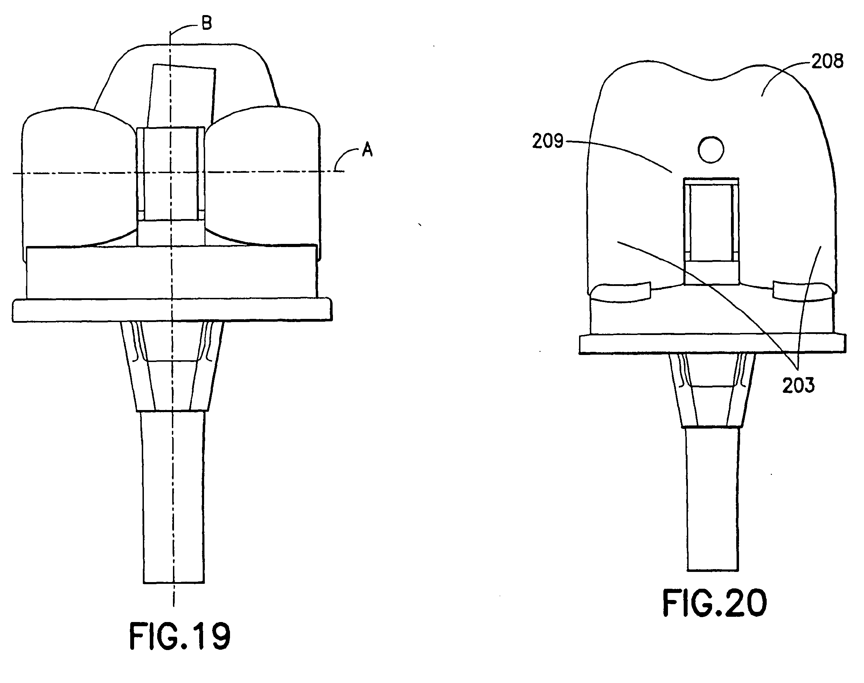

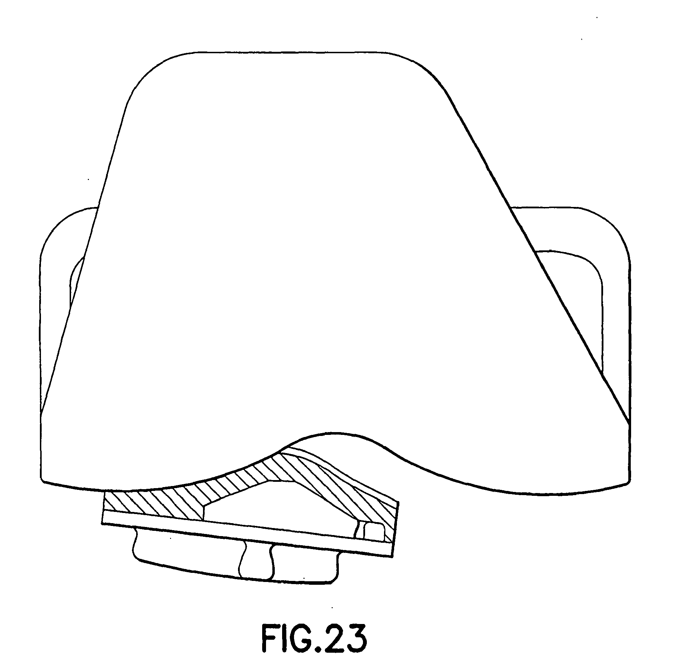

The femoral body 201 includes a patellofemoral articulating surface 207 with a superior region 208 and an inferior region 209 as shown in FIGS. 13 and 20. The superior region 208 of the patellofemoral articulating surface 207 is wider than the inferior region 209 since at lower flexion angles the patella 900 is in a relatively superior position, and may be displaced medially, as shown in FIG. 23. At moderate to large flexion, the patella 900 is central and the inferior patellofemoral articulating surface 209 need not be wider than the patella 900.

The prosthesis 100 is implanted by assembling an appropriate femoral stabilizing rod 202 to the femoral body 201 as described for example, in U.S. Patent No. 5,074,879. The plastic bushings 210 also are mounted in the holes 206 of the femoral body 201. As a result, the thrust flanges 211 of the plastic bushings 210 limit the insertion of the plastic bushings 210 into the holes 206. Additionally, the substantially cylindrical hinge bearing surfaces 212 are located centrally in the holes 206. This subassembly of the femoral body 201, the femoral stabilizing rod 202 and the plastic bushings 210 define the femoral component 200.

The bearing 400 then is assembled with the carriage 310 of the hinge subassembly 300. More particularly, the shaft 313 of the carriage 310 is inserted into the hole 402 of the bearing 400. The snap ring 409 engages in the groove 507 to prevent unintended separation of the carriage 310 from the bearing 400. The pin support hole 315 of the carriage 310 then is aligned with the hinge bearing surface 212 in the bushing 210 of the femoral component 200. The hinge pin 320 then is passed into a first hinge bearing surface 212, through the support hole 315 of the carriage 310 and into the second hinge bearing surface 212. The set screw 330 then is introduced into the threaded hole 314. As the set screw 330 is tightened, the conical end 331 thereof engages in the groove 322 in the hinge pin 320, thereby clamping the pin 320 in place. This clamping is important to avoid metal-to-metal micro-motion that could generate harmful metallic wear debris. The tibial body 501 and the tibial stabilizing rod 502 then are assembled to form the component 500.

The tibia and the femur are prepared in a known manner, including forming channels to receive the stabilizing rods 202 and 502 respectively. A box-like cavity is prepared in the central, distal and posterior aspect of the femur. The cavity is dimensioned to define an envelope surrounding the two support walls 204. The tibial component 500 then is implanted into the tibia and the femoral component 200 and hinge subassembly 300 are implanted in the femur 600. The joint then is distracted and the tapered end of the cone 407 of the bearing 400 is inserted into the conical hole 503 of the tibial body 501. The joint then is closed so that the implanted prosthesis 100 is in the disposition shown in FIGS. 1 and 19-23. The assembled prosthesis permits rotation about the axis A and the axis B in FIG. 19.

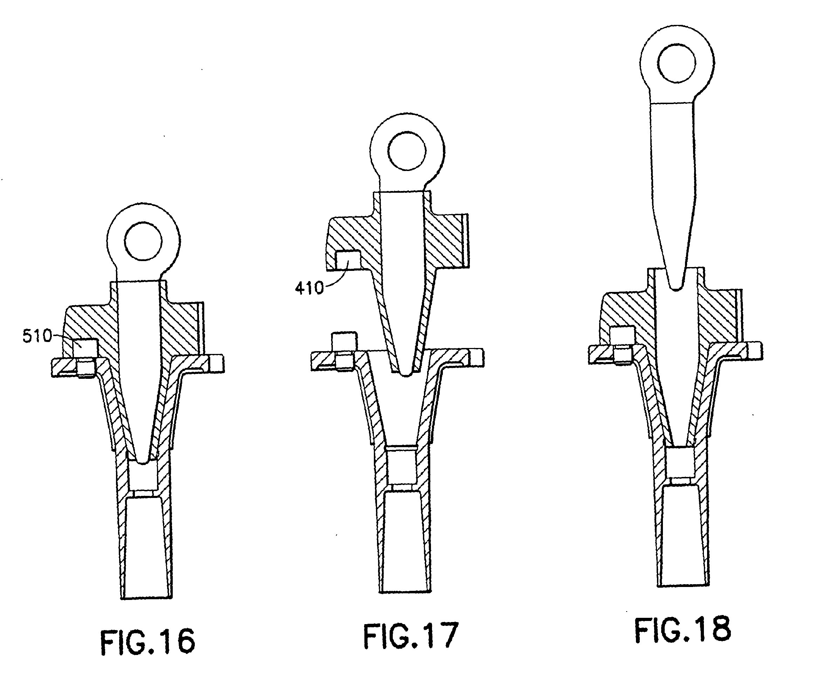

The prosthesis 100 provides several advantages. First, the prosthesis 100 provides the valgus-varus stability with full congruency through the complete anticipated range of tibiofemoral articulation. Additionally, the superior patellofemoral articulation still maintains patellar tilt at low to moderate flexion angles. A patient who requires a hinged prosthetic joint is likely to have collateral ligaments that are deficient or absent. Hence, the remaining natural components of the knee joint may not be sufficient to resist dislocation. However, the snap ring or other such retention mechanism between the bearing 400 and the tibial component 500 prevents the relatively small amount of distraction shown in FIG. 17 that could lead to dislocation. Rather, the much larger distraction shown in FIG. 18 would be required to achieve by completely separating the much longer shaft 313 of the carriage 310 from the bearing 400. Accordingly, the knee prosthesis 100 provides very good dislocation resistance with a relatively short cone on the tibial component 500.

While the invention has been described with respect to certain embodiments, it is apparent that various changes can be made without departing from the scope of the invention as defined by the appended claims.

## Claims

1. A hinged knee prothesis (100) comprising:
a tibial component (500) having an inferior surface for mounting to a tibia and a superior bearing surface (504),
a bearing (400) having an inferior bearing surface (406) supported on the tibial component and a superior condylar bearing surface (401),
a hinge carriage (310) having a shaft (313) mounted in the bearing (400) and a head (311); and
a femoral component (200) having a superior surface for mounting to a femur, an inferior tibiofemoral articular surface (203) configured relative to the condylar bearing surface (401) for achieving congruent tibiofemoral articulation, the femoral component (200) further having a patellofemoral articulating surface (207) distinct from the tibiofemoral articular surface (203) for maintaining a substantially centralized disposition of the patella at large flexion angles, **characterised in that** the patellofemoral articulating surface (207) is adapted for permitting patellar tilt at low to moderate flexion angles,
wherein the patellofemoral articulating surface (207) includes a superior region (208) and an inferior region (209), the superior region (208) of the patellofemoral articulating surface (207) being wider than the inferior region (209) thereof to permit medial displacement of the patella at low flexion angles.

2. The prothesis of claim 1, wherein the tibiofemoral articular surface (203) of the femoral component (200) is configured relative to the condylar bearing surface (401) of the bearing (400) to achieve fully congruent tibiofemoral articulation at least through a range from full extension to at least 90° flexion.

3. The prosthesis of claim 1 or 2, wherein fully congruent tibiofemoral articulation is achieved to approximately 150° flexion.

4. The prosthesis of any claims 1 to 3, further including a stop surface on the femoral component (200) and a stop surface (408) on the bearing (400) that engage and limit hyperextension of the prothesis.

5. The prothesis of any of claims 1 to 4, wherein the tibial component (500) includes a conical hole (503) extending into a superior surface (504) thereof, the bearing (400) including a cone (407) pivotally engaged in the conical hole (503) of the tibial component (500) for rotation about central axes of the cone (407) and the conical hole (503).

6. The prothesis of claim 5, further comprising engagement means between the cone (407) of the bearing (400) and the conical hole(503) of the tibial component (500) for preventing axial separation and thereby resisting dislocation of the prothesis.

7. The prothesis of claim 5 or 6, wherein the engagement means includes an annular groove (507) formed in the conical hole (503) of the tibial component (500) and a projection formed on the cone (407) of the bearing (400).

8. The prothesis of any claims 5 to 7, wherein the cone (407) of the bearing (400) includes an annular groove (409) and the projection is a snap ring engaged in the annular grooves of the cone (407) of the bearing (400) and the conical hole (503) of the tibial component (500).

9. The prosthesis of any of claims 1 to 8, wherein the femoral component (200) includes two spaced apart hinge support walls (204) disposed respectively on opposite respective sides of the head of the carriage (310) for resisting valgus-varus moments.

## Patentansprüche

1. Gelenkige Knieprothese (100) umfassend:
eine tibiale Komponente (500) mit einer unteren Fläche zum Anbringen an einer Tivia und einer oberen Lagerfläche (504),
ein Lager (400) mit einer unteren Lagerfläche (406), die an der tibialen Komponente gehalten wird, und einer oberen kondylären Lagerfläche (401),
ein Gelenkschlitten (310) mit einem Schaft (313), der im Lager (400) angebracht ist, und einem Kopf (311); und
eine femorale Komponente (200) mit einer oberen Fläche zum Anbringen am Femur, einer unteren tibiofemuralen Gelenkfläche (203) die relativ zur kondylären Lagerfläche (401) zum Erreichen einer kongruenten tibiofemoralen Gelenkverbindung konfiguriert ist, wobei die femorale Komponente (200) ferner eine von der tibiofemoralen Gelenkfläche (203) verschiedene patellofemorale Gelenkfläche (207) zum Aufrechterhalten der im Wesentlichen zentralen Anordnung der Patella bei großen Beugungswinkeln aufweist, **dadurch gekennzeichnet, dass** die patellofemorale Gelenkfläche (207) dafür ausgelegt ist, bei kleinen bis mäßigen Beugungswinkeln eine patellare Neigung zu gestatten,
wobei die patellofemorale Gelenkfläche (207) einen oberen Bereich (208) und einen unteren Bereich (209) beinhaltet, wobei der obere Bereich (208) der patellofemoralen Gelenkfläche (207) breiter als dessen unterer Bereich (209) ist, um eine mediale Versetzung der Patella bei geringen Beugungswinkeln zu gestatten.

2. Prothese nach Anspruch 1, bei dem die tibiofemorale Gelenkfläche (203) der femoralen Komponente (200) relativ zur kondylären Lagerfläche (401) des Lagers (400) so konfiguriert ist, dass eine vollständig kongruente tibiofemorale Gelenkverbindung wenigstens über einen Bereich von der vollständigen Streckung bis wenigstens 90° Beugung erreicht wird.

3. Prothese nach Anspruch 1 oder 2, bei der eine vollständig kongruente tibiofemorale Gelenkverbindung bis zu annähernd 150° Beugung erreicht wird.

4. Prothese nach einem der Ansprüche 1 bis 3, die ferner eine Anschlagfläche an der femoralen Komponente (200) und eine Anschlagfläche (408) am Lager (400) beinhaltet, die aneinander angreifen und eine Überstreckung der Prothese begrenzen.

5. Prothese nach einem der Ansprüche 1 bis 4, bei der die tibiale Komponente (500) ein konisches Loch (503) beinhaltet, das sich in deren obere Fläche (504) erstreckt, das Lager (400) einen Konus (407) beinhaltet, der mit dem konischen Loch (503) der tibialen Komponente (500) zur Drehung um die zentralen Achsen des Konus (407) und des konischen Lochs (503) schwenkbar im Eingriff ist.

6. Prothese nach Anspruch 5, ferner umfassend Eingriffsmittel zwischen dem Konus (407) des Lagers (400) und dem konischen Loch (503) der tibialen Komponente (500) zum Verhindern einer axialen Trennung und **dadurch** Widerstehen einer Dislokation der Prothese.

7. Prothese nach Anspruch 5 oder 6, bei der das Eingriffsmittel eine in dem konischen Loch (503) der tibialen Komponente (500) ausgebildete ringförmige Nut (507) und einen am Konus (407) des Lagers (400) ausgebildeten Vorsprung beinhaltet.

8. Prothese nach einem der Ansprüche 5 bis 7, bei der der Konus (407) des Lagers (400) eine ringförmige Nut (409) beinhaltet und der Vorsprung ein Sprengring ist, der mit den ringförmigen Nuten des Konus (407) und des Lagers (400) und dem konischen Loch (503) der tibialen Komponente (500) im Eingriff ist.

9. Prothese nach einem der Ansprüche 1 bis 8, bei der die femorale Komponente (200) zwei mit Abstand voneinander angeordnete Gelenkstützwände (204) beinhaltet, die jeweils auf entgegengesetzten jeweiligen Seiten des Kopfes des Schlittens (310) angeordnet sind, um Valgus-Varus-Momenten zu widerstehen.

## Revendications

1. Prothèse de genou à charnière (100) comprenant :
un composant tibial (500) ayant une surface inférieure pour montage sur un tibia, et une surface de portée supérieure (504),
une portée (400) ayant une surface de portée inférieure (406) supportée sur le composant tibial, et une surface de portée condylaire supérieure (401),
un chariot de charnière (310) ayant une queue (313) montée dans la portée (400), et une tête (311), et
un composant fémoral (200) ayant une surface supérieure pour montage sur un fémur, une surface articulaire tibio-fémorale inférieure (203) configurée par rapport à la surface de portée condylaire (401) pour obtenir une articulation tibio-fémorale congruente, le composant fémoral (200) ayant en outre une surface d'articulation patello-fémorale (207) distincte de la surface articulaire tibio-fémorale (203) pour maintenir une disposition sensiblement centrale de la rotule à de grands angles de flexion,
**caractérisée en ce que** la surface d'articulation patello-fémorale (207) est prévue pour permettre une inclinaison patellaire à des angles de flexion faibles à modérés,
dans laquelle la surface d'articulation patello-fémorale (207) comprend une région supérieure (208) et une région inférieure (209), la région supérieure (208) de la surface d'articulation patello-fémorale (207) étant plus large que sa région inférieure (209) pour permettre un déplacement médial de la rotule à de faibles angles de flexion.

2. Prothèse selon la revendication 1, dans laquelle la surface articulaire tibio-fémorale (203) du composant fémoral (200) est configurée, par rapport à la surface de portée condylaire (401) de la portée (400), de manière à obtenir une articulation tibio-fémorale complètement congruente au moins dans une plage allant de l'extension totale à une flexion d'au moins 90 degrés

3. Prothèse selon la revendication 1 ou 2, dans laquelle une articulation tibio-fémorale entièrement congruente est obtenue à environ 150 degrés de flexion.

4. Prothèse selon une quelconque des revendications 1 à 4, comprenant en outre une surface de butée sur le composant fémoral (200) et une surface de butée (408) sur la portée (400) qui viennent en contact et limitent l'hyper-extension de la prothèse.

5. Prothèse selon une quelconque des revendications 1 à 4, dans laquelle le composant tibial (500) comprend un trou conique (503) s'étendant dans sa surface supérieure (504), la portée (400) comprenant un cône (407) engagé de façon pivotante dans le trou conique (503) du composant tibial (500) pour rotation autour des axes centraux du cône (407) et du trou conique (503).

6. Prothèse selon la revendication 5, comprenant en outre un moyen d'enclenchement entre le cône (407) de la portée (400) et le trou conique (503) du composant tibial (500) pour empêcher une séparation axiale et donc résister à un déboîtement de la prothèse.

7. Prothèse selon la revendication 5 ou 6, dans lequel le moyen d'enclenchement comprend une gorge annulaire (507) formée dans le trou conique (503) du composant tibial (500) et une saillie formée sur le cône (407) de la portée (400).

8. Prothèse selon une quelconque des revendications 5 à 7, dans laquelle le cône (407) de la portée (400) comprend une gorge annulaire (409) et la saillie est un anneau élastique engagé dans les gorges annulaires du cône (407) de la portée (400) et du trou conique (503) du composant tibial (500).

9. Prothèse selon une quelconque des revendications 1 à 8, dans laquelle le composant fémoral (200) comprend deux parois de support de charnière mutuellement espacées (204) disposées respectivement sur des côtés respectifs opposés de la tête du chariot (310) pour s'opposer aux moments valgus-varus.
